# EUROPEAN PATENT APPLICATION

(11) **EP 4 652 942 A1**
(43) Date of publication of application: **26.11.2025**
(21) Application number: 25178623.2
(22) Date of filing: 23.05.2025
(51) Int. Cl.: A61B 17/17, A61B 17/80

(54) **IMPLANT, DRILLING GUIDE, MODULAR SYSTEM**

(30) Priority: 23.05.2024 DE 102024114531
(71) Applicant: Biosurgex, S.L., 35001 Las Palmas de Gran Canaria (ES)
(72) Inventor: Petazzoni, Massimo, 20068 PESCHIERA BORROMEO, MILANO (IT); Monopoli Roca, Angelo, 35001 LAS PALMAS DE GRAN CANARIA (ES); Monopoli, Donato, 35001 LAS PALMAS DE GRAN CANARIA (ES)
(74) Representative: DREISS Patentanwälte PartG mbB

(57) **Abstract**

Implant for use in Tibial Plateau Leveling Osteotomy Surgery (TPLO), in particular for a dog or a cat, wherein the Implant (10) is attachable to the tibial corpus, in particular to a medial surface (58) of the tibia (20), and the tibial head (44) after circular-segment like cutting and rotating a caudal fragment (46) by screws (62a, 62b) through screw holes (64a, 64b) that extend along a screw hole axis (a, a1, a2, a3) from a medial surface (12) to a lateral surface (14), comprising a proximal head portion (24),a cranial extension (28) and a caudal extension (26).

## Description

The invention relates to an implant for use in Tibial Plateau Leveling Osteotomy (TPLO) surgery, a drilling guide guiding and positioning a drill at the tibia and a modular system comprising a said implant.

Tibial Plateau Leveling Osteotomy (TPLO) surgery is a specialized orthopedic procedure designed especially to treat cranial cruciate ligament (CCL) injuries in dogs, which is analogous to the anterior cruciate ligament (ACL) injuries in humans. The CCL plays a crucial role in stabilizing the knee joint, and its total or partial rupture can lead to pain, lameness, and long-term joint problems such as osteoarthritis.

TPLO surgery aims to reconfigure the dynamics of the dog's knee so that the torn or weakened ligament becomes unnecessary for stabilizing the joint during weight bearing. This is achieved by making a circular cut in the tibia and rotating the top segment, known as the tibial plateau, until its angle with respect to the rest of the tibia is changed. The tibial plateau comprises the lateral and medial tibial condyle. The repositioned tibial plateau is then stabilized with an implant and screws, allowing the bone to heal in its new configuration.

In WO 2022/182987 A1 an orthopedic spacer assembly including a bone plate and a spacer for TPLO is shown.

US 2021 021 27 38 describes a TPLO plate designed to stabilize the stifle joint in animals following cranial cruciate ligament injuries.

US 11,317,953 covers a TPLO bone plate that incorporates one or more suture eyelets to enhance the rotational stability of the joint or bone and facilitate the reattachment of soft tissue to the plate at anatomical locations.

The objective of the invention is to improve the state of the art in TPLO in terms of reattaching and stabilizing the cut segment of the tibial head to the tibia.

The objective is solved by an implant for use in Tibial Plateau Leveling Osteotomy Surgery (TPLO), in particular for a dog or a cat, according to claim 1. The implant is attachable to the tibial corpus, in particular to a medial surface of the tibia, and the tibial head after circular-segment like cutting and rotating a caudal fragment by screws through screw holes that extend along a screw hole axis from a medial surface to a lateral surface. The cut is achieved by a circular saw blade with a radius. The cut caudal fragment of the tibial head comprising at least the lateral and medial tibial condyle, preferred the whole tibial plateau.

The Implant comprises:
- a proximal head portion attachable to the caudal fragment,
- a cranial extension in distal and/or cranial direction is provided on a cranial side of the head portion,
- a caudal extension in distal direction is provided on a caudal side of the head portion.

The cranial extension of the implant is attachable to a medial-cranial surface of the tibial corpus and/or a medial-cranial surface of a not cut cranial tibial head fragment. The caudal extension of the implant is attachable to a medial surface of the tibial corpus.

This implant provides an improved stability and re attachment of the cut and rotated caudal fragment resulting in better healing of the animal. It has shown that the patient recovers faster from a TPLO when an implant according to this invention is used, than with conventional implants. Also by providing two extensions, the overall length of the implant in proximal-distal direction is shorter than state of the art implants for TPLO needing a smaller incision into the patient and being less invasive to the surrounding tissue.

Preferably, at the proximal head portion both the caudal extension and the cranial extension are provided, wherein the two extensions are spaced apart from each other. Further, the implant is preferably U- or V- or hook-shaped, wherein the two branches are formed by the extensions.

The cut can be performed with saw blades with various radii. Preferably, the implant is fitted to the radius. One implant per each saw blade and respective radius could be provided. It could be a specific method to do the planning of the TPLO surgery wherein the blade size is chosen before surgery and therein the respective fitting implant. There could be nine blade radii, for instance but not limited to 10, 12, 15, 18, 20, 21, 27, 30 and 33mm, wherein implants could have a laser marking that matches the radius of the planned cut. Further, it is beneficial if right hand implants for the right knee joint and left hand implants for the left knee joint are provided.

The head portion could have an arch-like configuration, enclosing an angle of about 180° and comprising a first and a second end portion, wherein the cranial extension is adjoined to the first end portion and the caudal extension is adjoined to the second end portion. The end portions could reach the cutting line of the tibia when the implant is attached to the tibia.

It is conceivable, that the caudal extension has a first section being adjoined to the head portion and being at least largely parallel to the cranial extension. With this, a better fit to the tibial anatomy can be achieved. It could be also not parallel, for instance the cranial extension could be extending in a more cranial direction depending on the anatomical conditions of the patient.

It is beneficial, if the caudal extension has a second section adjoining the first section, wherein the second section is distant from the proximal head portion, wherein the first section and the second section are located along different straight lines, lying on a circular track and/or include an obtuse angle. The obtuse angle is advantageously in the range of 100° to 140°.

It has proven to be advantageous if the caudal extension has a greater distal extension than the cranial extension.

It is conceivable that the implant is produced using a 3d-print process and wherein at least two screw holes are provided with a thread structure of a teardrop-like shape with a tip, wherein the tips of all holes are pointing in the same distal direction. The thread structure can be designed as a real thread or preferably as a thread structure consisting of largely parallel thread elements.

A circular marking with a radius cloud be provided on a medial surface of the implant, wherein the radius is largely the same than the radius of the circular segment-like cut. Such marking could be provided in between the head portion and extensions or on the extensions.

Beneficially the implant comprises at least one screw hole in each of the head portion, the caudal extension and the cranial extension. It is further beneficial if at least one screw hole is provided on the head portion, wherein at least one screw hole is provided with a thread structure.

In addition, it is conceivable when at least one screw hole is provided in the cranial extension and/or in the caudal extension, wherein at least one screw hole is provided with a thread structure or a ramp structure. Moreover, three screw holes could be provided on the head portion, three screw holes are provided on the caudal extension and two screw holes are provided on the cranial extension. Also, three screw holes in the head portion, two screw holes in the caudal extension and one screw hole in the cranial extension cloud be provided with a thread structure.

It has proven to be advantageous if one screw hole in the cranial extension and/or one screw hole in the caudal extension are provided with a ramp structure.

Further it is advantageous if each ramp structure is provided with a slope extending along a slope axis, wherein the slope axis each is directed to an anatomical center point that is at an anatomical point of the center point of the circular segment-like cut with which the head of the tibia is cut, wherein the slope axis intersects at this anatomical center point.

The slopes of the ramps are advantageously configured in such a way that when a screw is inserted into the screw hole with the ramp and tightened, a screw head of the screw brought into contact by the tightening exerts a force with a force direction through the anatomical center point or through a center point axis that is directed through the anatomical center point from medial to lateral. This results in a better compression of the caudal fragment to the tibia, thus resulting in better fixation and rehabilitation.

In the head portion one cranial screw hole, one caudal screw hole and one proximal screw hole between the cranial and caudal screw hole could be provided, wherein the cranial screw hole axis encloses an angle with the sagittal plane in proximal-distal direction in the range of 97-87° and preferred 92°, wherein the proximal screw hole axis encloses an angle with the sagittal plane in proximal-distal direction in the range of 89-79° and preferred 84°, wherein the caudal screw hole axis encloses an angle with the sagittal plane in proximal-distal direction in the range of 92-82° and preferred 87°. These angles in to the colloquial term vertical direction (proximal-distal-direction) offer a better attachment to the tibia by the screws and reduce the risk of a pullout of the bone structure of the tibia. Further, these angles ensure as far as possible that the screws will not invade the articular surfaces and are fixed towards areas of the tibia with largely sufficient bone stock.

In the head portion one cranial screw hole, one caudal screw hole and one proximal screw hole between the cranial and caudal screw hole could be provided, wherein the cranial screw hole axis encloses an angle with the sagittal plane in caudal-cranial direction in the range of 91-105° and preferred 96° or 100°, wherein the proximal screw hole axis encloses an angle with the sagittal plane in caudal-cranial direction in the range of 88-98° and preferred 93°, wherein the caudal screw hole axis encloses an angle with the sagittal plane in caudal-cranial direction in the range of 82-67° and preferred 77° or 72°. These angles in to the colloquial term horizontal direction (caudal-cranial-direction) offer a better attachment to the tibia by the screws and reduce the risk of a pullout of the bone structure of the tibia. Further, these angles ensure as far as possible that the screws will not invade the articular surfaces and are fixed towards areas of the tibia with largely sufficient bone stock.

Favorably the shape of the lateral surface is of organic structure, according to the tibia where it is to be attached. This reduces the need of bending the implant into the desired shape for a good fit to the medial surface of the tibia.

A drilling guide for guiding and positioning a drill while drilling at the tibia also addresses the objective of the invention. The drilling is provided for attaching the implant according to the invention with a non-head locking screw through one of the screw holes provided with a ramp structure. The drilling guide comprising:
- a bolt with a thread section and an attachment section to be bolted into a screw hole provided with a thread located in the proximity of the screw hole with a ramp structure, and
- a drill guide piece with a guiding section for a drill attachable to the bolt in that the drill is guided perpendicular to the screw hole with the ramp structure when the drill guide is attached to the bolt.

The attachment section is preferably round in that the drill guide piece is omnidirectional attachable to the attachment section.

With the drilling guide, drilling holes for screws improves the safety and recovery for the patient.

A modular system also addresses the objective of the invention. The modular system comprises a plurality of implants according to the invention. The modular system could be further comprising a drilling guide according to the invention. The plurality of implants could differ in sizing for the dog breed the surgery is to be performed on and/or the blade radius used to perform the cutting of the tibial head. Further, it is beneficial if right hand implants for the right knee joint and left-hand implants for the left knee joint are provided in the modular system.

A combination of some of the above mentioned features will result in a beneficial implant. With the two extensions the implant allows for craniodistal as well as distal compression at the same time. Due to its geometry, the implant is shorter compared to the state of the art and available implants on the market. Thus, the implant is more robust in counteracting rotational forces and allows for effective craniodistal compression. The fact that it is highly anatomical, has laser marking, or has a plate for each blade size is further beneficial.

The overall geometry of the implant makes it much more robust, more stable, and shorter compared to the state of the art for the same type of implants for TPLO surgical procedure. The implant allows for two compressions, in terms of directions, instead of one in previous implants: the first compression being craniodistal (cranial) and the second one being proximodistal (distal). Another advantage of this implant is that the screws can be widely spaced apart from each other, minimizing the plowing effect, because the leverage arm in counteracting forces is wide. The pull out risk of the screws is minimized.

With the implant, the drill guide and the modular system TPLO surgery can be advantageously improved on a variety of dog breeds from a small dog breed like a Chihuahua to a large dog breed like a Spanish Bulldog.

Further details and advantageous embodiments of the invention can be found in the following description, on the basis of which embodiments of the invention are described and explained in more detail.

Showing:
- Figure 1 and 2:: An Implant from different perspectives;
- Figure 3:: a circular saw blade cutting the tibia head;
- Figure 4:: the implant according to the fig. 1 and fig. 2, positioned on the tibia head cut according to fig. 3 and the tibial corpus;
- Figure 5:: a drilling guide attached to the implant according to fig. 4 a drill inserted;
- Figure 6:: the implant according to fig. 4 and 5 partly attached to the tibia corpus and the cut tibia head;
- Figure 7 and 8:: the implant with a schematic cut and the anatomical center of the cut from different perspectives;
- Figure 9:: angles of the screw hole axes to the sagittal plane form a caudal perspective; and
- Figure 10:: angles of the screw hole axes to the sagittal plane form a proximal perspective.

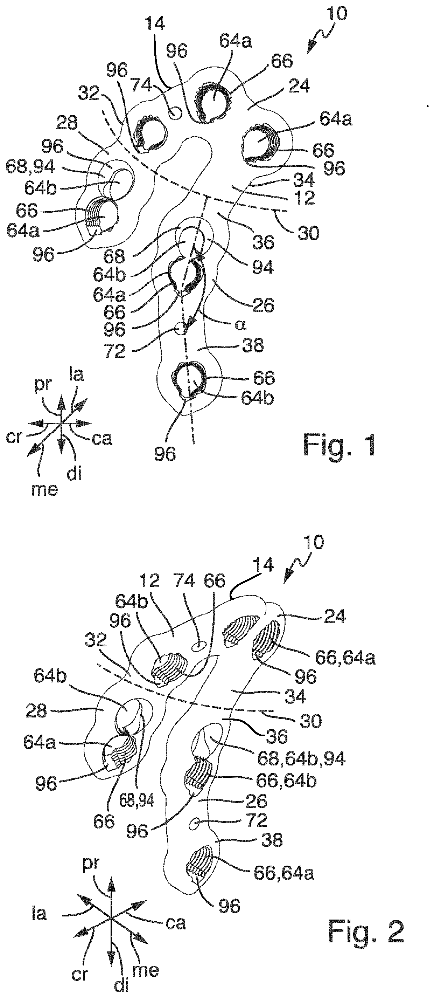

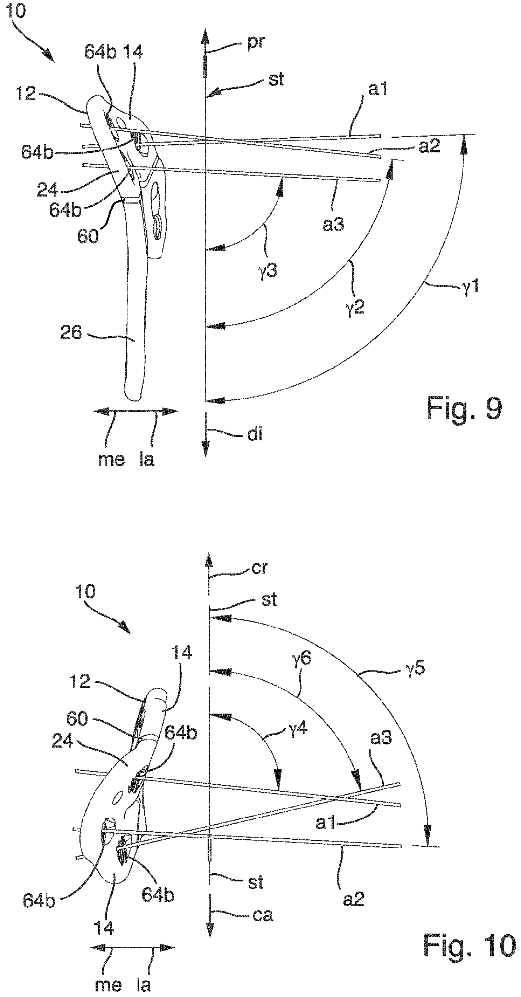

An Implant 10 for use in a Tibial Plateau Leveling Osteotomy Surgery (TPLO) is shown in fig. 1 and fig. 2 from different perspectives. Fig. 1 shows the implant 10 from a medial perspective and fig 2 shows the implant from a more cranial-proximal perspective compared to fig. 2. This particular embodiment is provided for a right knee joint of a dog, but all the following description is also applicable to a left side embodiment of the implant 10. Also, embodiments for other animals are possible. The implant 10 is of a flat shape having a medial surface 12 and a lateral surface 14, whereby the lateral surface is better shown in fig. 9 and fig 10.

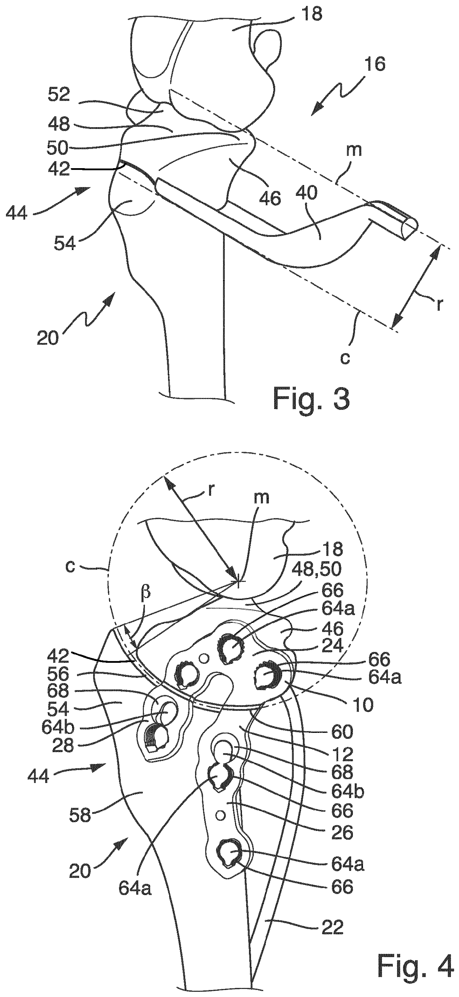

In fig. 1 and fig. 2, a system of coordinates for a better reference of the implant 10 with the directions proximal pr, distal di, cranial cr, caudal ca, lateral la and medial me, is shown. The orientation of fig. 1 is also valid for the following figure 4 and the orientation of fig. 2 is valid for figures 3, 5 and 6, wherein figures 3 to 6 showing a right hand side knee joint 16 with the femur 18 and the tibia 20. In figure 4 also the fibula 22 is shown.

With figures 1 and 2 the general shape of the implant 10 shall be explained. The implant 10 consists of a proximal head portion 24, a cranial extension 26 and a caudal extension 28. The cranial extension 26 and a caudal extension 28 are separate to each other and are connected to each other by the head portion 24. A possible imaginary boarder 30 of a dotted line shows the region where the head portion 24 ends and where the extensions 26, 28 begin.

The head portion 24 has an arch-like configuration, enclosing an angle of about 180° and comprising a first 32 and a second end portion 34, wherein the cranial extension 28 is adjoined to the first end portion 32 and the caudal extension 26 is adjoined to the second end portion 34. The caudal extension 26 has a first section 36 being adjoined to the head portion 24 and being at least largely parallel to the cranial extension 28. Further, the caudal extension 26 has a second section 38 adjoining the first section 36 and distant from the proximal head portion 24, wherein the first section 36 and the second section 38 include an obtuse angle α. It is also like this, that the first section 36 and second section 38 form a circular track as the overall shape of the implant 10 is organic. It can be seen, that the caudal extension 26 has a greater distal extension than the cranial 28 extension.

Furthermore, the implant can be described as U- or V- or hook-shaped, wherein the two separate branches are formed by the extensions 26, 28.

The implant 10 has various screw holes 64a, 64b, which each extend from the medial surface 12 to the lateral surface 14 each along a screw hole axis a; the configuration of various screw hole axes is explained further below with figures 9 and 10.

Figure 3 shows the knee joint 16 with a circular saw blade 40 with a radius r between the cutting line c and its center point axis m from a medial perspective. Usually in a TPLO surgery the approach is made from a medial direction. The saw blade 40 is used to perform a circular cut 42 into the tibial head 44 to separate a caudal fragment 46 from the tibial head 44 comprising the tibial plateau 48 comprising medial tibial condyle 50 and lateral tibial condyle 52. The cut 42 separates the tibial head 44 into said caudal fragment 46 and a not cut cranial tibial head fragment 54. The cut 42 has the same radius r as of the used circular saw blade 40.

The cut 42 can be performed with saw blades 40 with various radii. The later used implant 10 fits to the radius. One implant 10 per each saw blade 40 and respective radius r could be provided in a set or a set of a saw blade. It could be a specific method to do the planning of the TPLO surgery wherein the blade size (radius r) is chosen before TPLO surgery and therein the respective fitting implant 10.

There could be nine blades radii r, for instance but not limited to the radius r could be 10, 12, 15, 18, 20, 21, 27, 30 and 33mm as it has shown that these radii fit to the most common dog breeds. The implants 10 could have a laser marking that matches the radius r of the planned cut 42.

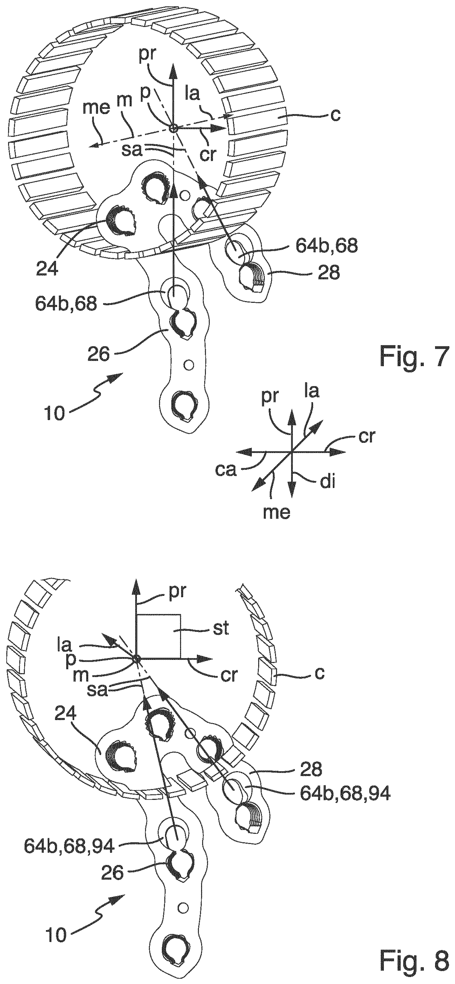

Further, it is beneficial if right hand implants 10 for the right knee joint 16 and left hand implants 10 for the left knee joint 16 are provided. Right hand implants 10 are shown in figures 1, 2, 4, 5, 6, 9 and 10. Left hand implants 10 are shown in figures 7 and 8.

Fig. 4 shows the tibia 20 with a gap 56 between the caudal fragment 46 and the cranial fragment 52, where the cut 42 was performed according to figure 3. The cut 42 lays on the circular cutting line c with the center point on the center point axis m with the radius r, the circle c has the same radius r as the circular saw blade 40.

The caudal fragment 46 is rotated around the center point m in caudal direction by the angle β. The implant 10 is roughly positioned on the medial surface 58 of the tibia 20 with at least partially contacting the tibia with its lateral surface 14. The implant 10 is not yet attached to the tibia 20. It can be seen that the implant 10 is yet not in its final position as a marking 60 of the implant 10 on its medial surface 12 that matches the radius r is not yet fully positioned on the cut 42. Additional to the marking 60 the radius r in millimeters could be written on the implant.

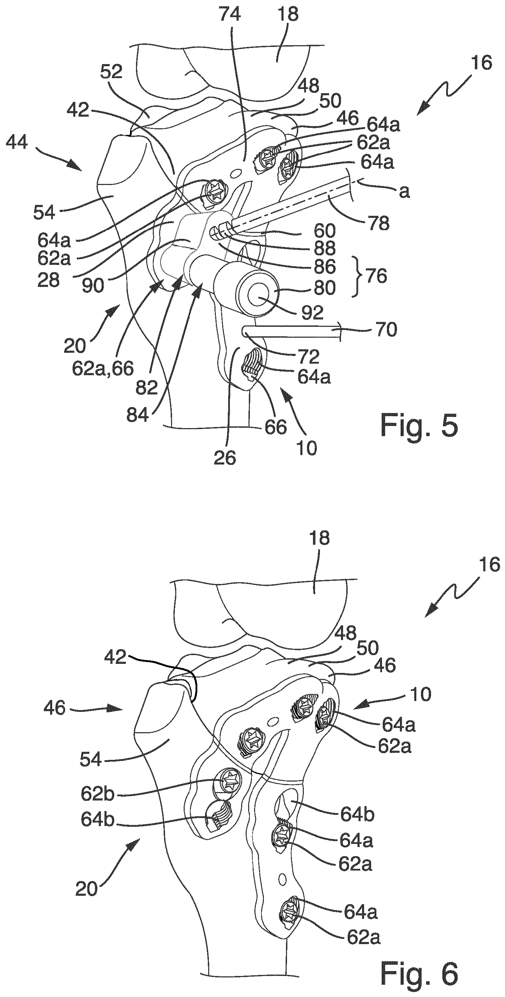

Figures 5 and 6 show the attachment of the implant 10 to the tibia by screws 62a, 62b through screw holes 64a, 64b. For the attachment different head locking screws 62a and non-head locking screws 62b are provided. Respective screw holes 64a, 64b for the screws 62a, 62b are provided, wherein screw holes 64a with a thread structure 66 are provided for the head locking screws 62a and screw holes 64b with a ramp structure 68 are provided for the non-head locking screws 62b. The different screw holes 64a, 64b are also shown in the other figures of the implant 10, especially in figures 1 and 2.

In the embodiment according to figures 1, 2, 4-10, the implant 10 shows three screw holes 64a with a thread structure 66 in the head section 24. One screw hole 64a is provided at a distal location of the cranial extension 28 and two screw holes 64a in the caudal extension 26, wherein one screw hole 64a is provided at a distal location of the first section 36 and one at a distal location of the second section 38. It is preferred if the screw holes 64a with a thread structure 66 are conical.

Referring to the screw holes 64b with the ramp structure 68, there is each one screw hole 64b provided in each of the caudal extension 26 and cranial extension 28. The screw hole 64b of the caudal extension 26 is provided in the first section 36.

In this embodiment, best shown in figures 1 and 2, each screw hole 64b with a ramp structure 68 is connected with the closest screw hole 64a with a thread structure 66, in that the two screw holes 64a, 64b are forming an eight shaped hole. The screw hole 62a is located in a distal direction to the screw hole 64b.

However, there could be other configurations of screw holes 64a, 64b conceivable, for example only two or four screw holes 64a in the head section 24, more than one screw hole 64b in each of the extensions 26, 28 and more screw holes 62a in the extensions 26, 28. In addition, other locations of the overall screw holes 64a, 64b is possible depending on the anatomical situation for the desired TPLO.

In figure 5 there is also a k-wire 70 inserted into a k-wire hole 72 in the caudal extension 26 through the implant 10 into the tibia 20 to stabilize the implant 10 and hold it into position while not fully attached to the tibia 20 by screws 62a, 62b. A second k-wire hole 74 is provided in the head section 24. In addition, more k-wire holes could be provided in the implant 10.

For attachment of the implant 10, first three head locking screws 62a are inserted in each into the provided screw holes 64a after a hole was drilled into the caudal fragment 46. The screws 62a are each tightened until the head locking part each is each inserted into the thread structure 66 attaching the implant 10 securely to the caudal fragment 46.

After attaching the head section 24 of the implant 10 by tightening the three screws 62a in the head section 24, holes are drilled into the tibia 20 the non-head locking screws 62b. To drill suitable holes, a drilling guide 76 for guiding and positioning a drill 78 is used.

The drilling guide 76 comprises a bolt 80 with a thread section 82 and an attachment section 84 and further a drill guide piece 86 with a guiding section 88 for the drill 78. The bolt 80 is bolted into the screw hole 62a with the thread structure 66 of the cranial extension 28 with its thread section 82. The drill guide piece 86 is attached to the attachment section 84. The attachment section 84 is round, in that the drill guide piece 86 is omnidirectional attachable to the bolt 80. The drill guide piece 86 is removable attachable to the bolt 80 by a clip type fastening section 90. The guiding section 88 is formed like a cylindrical hole, with a diameter according to the drill 78 used, through the whole drill guide piece 86. The drill guide 76 is configured in that the guiding section 88 is in line with the screw hole axis a of the respective screw hole 64b, thus aligning the drill 78 with the screw hole axis a. This makes drilling the holes for the non-head locking screws 62b easier and safer, as attaching a drill guide securely to the screw holes 64b with the ramp structure 68 is not possible.

In the bolt 80, a further drill guide section 92 to guide the drill 78 while drilling holes into the tibia 20 for the head locking screws 62a can be provided.

It is conceivable to provide multiple bolts 80 and/or drill guide pieces 86 to guide drills 78 with different diameters.

Further the bolt 80 and the drill guide piece 86 can be one-piece.

In figure 6 the knee joint 16 with the implant 10 is shown after the screws 62a, 62b were tightened. Especially the non-head locking screw 62b was inserted and tightened in the screw hole 64b of the cranial extension 28 resulting in a compression of the caudal fragment 46 to the tibia 20 along the cut 42 wherein the gap 56 (fig. 4) is no longer present.

The compression results from tightening the non-head locking screw 62b in the screw hole 64b against the ramp structure 68. The ramp structure 68 is provided with a slope 94 directed along a slope axis sa (fig. 7, 8) in proximal direction. By tightening the screw 64b, the head of the screw 64b presses against the ramp section 68 and sliding downwards the slope 94, moving the implant 10 with the attached caudal fragment 46 into a distal or distal-cranial direction. This movement continues until the screw head comes to rest at a proximal end of the screw hole 64b, because the screw holes 64b are configured in a slot like shape. The movement of the implant 10 and with it the attached caudal fragment 46 results in a force f along the respective slope axis sa of the screw holes 64b.

The working principle of the compression of the screw holes 64b with the ramp structure 68 and the non-head locking screws 62a is explained better with figures 7 and 8 as the bone structure of the knee joint 16 is not shown there. The cutting line c in medial-lateral direction, the center point axis m and the implant 10 are shown respectively. Further, an anatomical center point p of the cut 42 within the joint 16 is shown. Here it can be seen that the forces f run along the axes sa, wherein the slope axes intersect sa at the anatomical center point p. Therefore, each of the forces f are acting on the anatomical center point p. The slope axes sa are preferably arranged in that they are not coplanar to each other, and thereby also not the forces f. In figure 8 a plane st parallel to the anatomical sagittal plane is added for reference of the orientation of the implant 10.

In figures 9 and 10 a further aspect of the implant 10 is shown. In order to improve attachment of the head section 24 to the caudal fragment 46, resulting in a better compression of the caudal fragment 46 to the tibia 20, the axes of the three screw holes 64a provided in the head portion 24 are configured in certain angles to the sagittal plane or a parallel plane st to the sagittal plane.

Figure 9 shows the implant from a caudal-cranial direction, wherein for all screw holes 62a provided in the head portion 24 the respective screw hole axes a1, a2, a3 are shown. The screw hole axis a1 of the cranial screw hole 64a encloses an angle γ1 with the plane st in distal direction of cranial screw hole axis encloses an angle with the sagittal plane in of 92°, wherein the angle γ1 can be in the range of 97-87°. The screw hole axis a2 of the proximal screw hole 64a encloses an angle γ2 with the plane st in distal direction of 84°, wherein the angle γ2 can be in the range of 89-79°. The screw hole axis a3 of the caudal screw hole 64a encloses an angle γ3 with the plane st in distal direction of 87°, wherein the angle γ3 can be in the range of 92-82°.

Figure 10 shows the implant from a proximal-distal direction. For all screw holes 62a provided in the head portion 24 the respective screw hole axes a1, a2, a3 are shown. The screw hole axis a1 of the cranial screw hole 64a encloses an angle γ4 with the plane st in cranial direction of 96° or 100°, wherein the angle can be in the range of 91-105°.The screw hole axis a2 of the proximal screw hole 64a encloses an angle γ5 with the plane st in cranial direction of 93°, wherein the angle can be in the range of 88-98°.The screw hole axis a3 of the caudal screw hole 64a encloses an angle γ6 with the plane st in distal wherein the caudal screw hole axis encloses an angle with the sagittal plane in plane st in cranial direction of 77° or 72°, wherein the angle can be in the range of 82-67°.

With the angles γ1, γ2, γ3, γ4, γ5, γ6 enclosing the said values or ranges a beneficial attachment of the implant 10 can be ensured.

The implant 10 can be made by rapid prototyping, especially metal 3D-printing with a biocompatible metal such as titanium. For an improved quality the thread structures 66 in the screw holes 64a can be configured of multiple stacked parallel elements, that resemble a thread, but are not of the usual thread with a thread pitch. Further, for improved production, the screw holes 64a with this thread structure 66 are of a teardrop-like shape with a tip 96, wherein the tips 96 of all holes are pointing in the same distal direction.

Overall, with an implant 10 and a drilling guide 76, or a modular system comprising multiple implants 10 TPLO surgery can be improved.

## Claims

1. Implant for use in Tibial Plateau Leveling Osteotomy Surgery (TPLO), in particular for a dog or a cat, wherein the Implant (10) is attachable to the tibial corpus, in particular to a medial surface (58) of the tibia (20), and the tibial head (44) after circular-segment like cutting and rotating a caudal fragment (46) by screws (62a, 62b) through screw holes (64a, 64b) that extend along a screw hole axis (a, a1, a2, a3) from a medial surface (12) to a lateral surface (14), comprising:
- a proximal head portion (24) attachable to the caudal fragment (46),
- a cranial extension (28) in distal and/or cranial direction is provided on a cranial side of the head portion (24),
- a caudal extension (26) in distal direction is provided on a caudal side of the head portion (24),
wherein the cranial extension (28)is attachable to a medial-cranial surface of the tibial corpus and/or a medial-cranial surface of a not cut cranial tibial head fragment,
wherein the caudal extension (26) is attachable to a medial surface of the tibial corpus.

2. Implant (10) according to claim 1, wherein the head portion (24) has an arch-like configuration, enclosing an angle of about 180° and comprising a first end portion (32) and a second end portion (34), wherein the cranial extension (28)is adjoined to the first end portion (32) and the caudal extension (26) is adjoined to the second end portion (34).

3. Implant (10) according to claim 2, wherein the caudal extension (26) has a first section (36) being adjoined to the head portion (24) and being at least largely parallel to the cranial extension (28).

4. Implant (10) according to claim 3, wherein the caudal extension (26) has a second section (38) adjoining the first section (36) and distant from the proximal head portion (24), wherein the first section (36) and the second section (38) are located along different straight lines, lying on a circular track and/or include an obtuse angle (α).

5. Implant (10) according to any of the proceeding claims, wherein the Implant (10) is produced using a 3-print process and wherein at least two screw holes are provided with a thread structure (66) and of a teardrop-like shape with a tip (96), wherein the tips (96) of all screw holes (64a) are pointing in the same distal direction.

6. Implant (10) according to any of the proceeding claims, wherein a circular marking (60) with a radius (r) is provided on a medial surface (12) of the implant (10), wherein the radius (r) is the same than the radius (r) of the circular segment-like cut (42).

7. Implant (10) according to any of the proceeding claims, comprising at least one screw hole (64a, 64b) in each of the head portion (24), the caudal extension (26) and the cranial extension (28).

8. Implant (10) according to any of the proceeding claims, wherein at least one screw hole (64a, 64b) is provided on the head portion (24), wherein at least one screw (64a) hole is provided with a thread structure (66).

9. Implant (10) according to any of the proceeding claims, wherein at least one screw hole (64a, 64b) is provided in the cranial extension (28)and/or in the caudal extension (26), wherein at least one screw hole (64a, 64b) is provided with a thread structure (66) or a ramp structure (68).

10. Implant (10) according to any of the proceeding claims wherein one screw (64b) hole in the cranial extension (28)and/or one screw hole (64b) in the caudal extension (26) are provided with a ramp structure (68).

11. Implant (10) according to claim 9 or 10, wherein each ramp structure (68) is provided with a slope (94) extending along a slope axis (sa), wherein the slope axis (sa) each is directed to a anatomical center point (p) that is at an anatomical point of the center point of the circular segment-like cut (42) with which the head of the tibia (44) is cut, wherein the slope axis (sa) intersect at this anatomical center point (p).

12. Implant (10) according to 10, wherein the slopes (94) of the ramps (68) are configured in such a way that when a screw (62b) is inserted into the screw hole (64b) with the ramp (68) and tightened, a screw head of the screw (62b) brought into contact by the tightening exerts a force (f) with a force direction through the anatomical center point (p) or through a mid point axis (m) that is directed through the anatomical center point (p) from medial to lateral.

13. Implant (10) according to any of the proceeding claims, wherein one cranial screw hole (64a), one caudal screw hole (64a) and one proximal screw hole (64a) between the cranial and caudal screw hole (64a) are provided in the head portion (24), wherein the cranial screw hole axis (a1) encloses an angle (γ1) with the sagittal plane (st) in proximal-distal direction in the range of 97-87° and preferred 92°,
wherein the proximal screw hole axis (a3) encloses an angle (γ2) with the sagittal plane (st) in proximal-distal direction in the range of 89-79° and preferred 84°,
wherein the caudal screw hole axis (a3) encloses an angle (γ3) with the sagittal plane (st) in proximal-distal direction in the range of 92-82° and preferred 87°.

14. Implant (10) according to any of the proceeding claims, wherein one cranial screw hole (64a), one caudal screw hole (64a) and one proximal screw hole (64a) between the cranial and caudal screw hole (64a) are provided in the head portion (24), wherein the cranial screw hole axis (a1) encloses an angle (γ4) with the sagittal plane (st) in caudal-cranial direction in the range of 91-105° and preferred 96° or 100°,
wherein the proximal screw hole axis (a2) encloses an angle (γ5) with the sagittal plane (st) in caudal-cranial direction in the range of 88-98° and preferred 93°,
wherein the caudal screw hole axis (a3) encloses an angle (γ6) with the sagittal plane (st) in caudal-cranial direction in the range of 82-67° and preferred 77° or 72°.

15. Drilling guide (76) for guiding and positioning a drill (78) while drilling at the tibia (20) for attaching the implant (10) according to one of the claims 10 to 14 with a non-head locking screw (62b) through one of the screw holes (64b) provided with a ramp structure (68), comprising
a bolt (80) with a thread section (82) and an attachment section (84) to be bolted into a screw hole (64a) provided with a thread structure (66) located in the proximity of the screw hole (64b) with a ramp structure (68) and
a drill guide piece (86) with a guiding section (88) for a drill (78) attachable to the bolt (80) in that the drill (78) is guided perpendicular to the screw hole (64b) with the ramp structure (68) when the drill guide piece (86) is attached to the bolt (80).

16. A modular system comprising a plurality of implants (10) according to at least one of the claims 1 to 14 and/or a drilling guide (76) according to claim 15.
